# EUROPEAN PATENT SPECIFICATION

(11) **EP 3 966 581 B1**
(45) Date of publication and mention of the grant of the patent: **10.07.2024**
(21) Application number: 20719660.1
(22) Date of filing: 24.04.2020
(51) Int. Cl.: G01R 33/34

(54) **RF COIL ASSEMBLY AND MRI APPARATUS**
HF-SPULENANORDNUNG UND MRT-VORRICHTUNG
ENSEMBLE DE BOBINE RF ET APPAREIL D'IRM

(30) Priority: 06.05.2019 WO PCT/CN2019/085661; 27.06.2019 EP 19182833
(43) Date of publication of application: 16.03.2022
(73) Proprietor: Koninklijke Philips N.V., 5656 AG Eindhoven (NL)
(72) Inventor: DENG, Wen, 5656 AE Eindhoven (NL); WU, Bing, 5656 AE Eindhoven (NL); HARVEY, Paul, Royston, 5656 AE Eindhoven (NL)
(74) Representative: Philips Intellectual Property & Standards
(86) International application number: PCT/EP2020/061456
(87) International publication number: WO 2020/224991

(56) References cited:
- WO-A1-2018/097863
- US-A1- 2008 007 250
- US-A1- 2008 143 333
- US-A1- 2014 191 757
- US-A1- 2018 097 274
- US-A1- 2018 136 293
- US-A1- 2018 306 877
- US-B1- 7 526 330

## Description

### FIELD OF THE INVENTION

The invention relates to a RF (radio frequency) coil assembly. More particularly, the present invention relates to a RF coil assembly adapted to be used with a magnetic resonance imaging (MRI) apparatus. The present invention also relates to a MRI apparatus comprising the RF coil assembly.

RF coil assemblies are used in MRI apparatus. In addition to "flat" coil assemblies, which are lightweight and would be used for MRI scans on a region of a subject such as the abdomen, there are "curved" coil assemblies, which take a non-planar form. The "curved" coil assemblies have typically a hard plastic housing for enclosing a flexible coil to hold the coil assembly in the desired configuration. The "curved" coil assemblies would be used for MRI scans on the region of the subject such as the head, the shoulder or the ankle where a substantially flat coil assembly is not appropriate. This kind of "curved" coil assemblies is usually cumbersome and fairly heavy. If the size and shape of a "curved" coil assembly required for a particular subject is not available, it can be time-consuming to replace a "curved" coil assembly for that subject. In addition, different "curved" coil assemblies are usually required for different "curved" regions of a subject such as the head, the shoulder or the ankle, which not only increases the cost but also results in difficulty in storage and management of the "curved" coil assemblies.

US2008/007250A1 describes an MRI RF coil array comprised of a large number of separate coil elements supported on a substrate shaped to the contour of the anatomy being imaged. However, it requires a contoured substrate and an array of polygonal tiles drawn on the surface of the contoured substrate to position the coil array on the substrate surface, which results in a complicated coil array manufacturing and also makes the positioning and fastening of the coil array to the anatomy burdensome. US7526330B describes an RF coil for imaging parts of a human head which includes upper and lower pairs of RF coils respectively supported by rigid lower and upper supporting structures. WO2019/097863A1 describes a mirror assembly for a RF head coil with a hard plastic housing. US2018/306877A1 mentions a positioner 1004 attached to a base 1002. US2018/136293A1 mentions the flexible local coil 300 is attached to the rigid frame 100 to form a tubular overall local coil. US2018/097274A1 discloses a RF coil comprising a flexible antenna circuit, a support that supports the flexible antenna circuit and a one-piece flexible casing enclosing the flexible antenna circuit 110 and the support 120.

Thus, there is a need to make improvements on the known non-planar "curved" coil assemblies.

### SUMMARY OF THE INVENTION

The present invention seeks to provide non-planar "curved" coil assemblies that are not only lighter and better match the regions of different subjects to be imaged than prior coil assemblies but are also applicable to different "curved" regions of the subject to be imaged.

According to one aspect of the present invention, there is provided a RF coil assembly as defined in claim 1.

According to the present invention, the RF coil assembly is not only lighter and better matches the regions of different subjects but it also may be used as a multiple purpose coil assembly that is applicable to different "curved" regions of the subjects. Furthermore, the RF coil assembly made of a flexible coil attached to a rigid frame can assume the desired shape and can be maintained therein for immediate use instead of having to achieve the desired shape by an operator attaching the flexible coil to body regions. Therefore, the positioning and fastening of the RF coil assembly is much easier and faster as compared to the conventional flexible coil, and the workflow of MRI scanning is enhanced.

According to one embodiment of the present invention, the shape of the RF coil assembly comprises a concave contour that conforms to a convex contour of the region of the subject and a half tubular contour that conforms to a half tubular contour of the region of the subject.. According to another embodiment of the present invention, the region of the subject comprises any one of anterior part of head, shoulder and ankle.. Advantageously, the concave contour portion and the half tubular contour portion of the flexible coil formed as a result of attaching the flexible coil to the first rigid frame can conform to multiple regions of a subject, thereby providing a multiple purpose RF coil assembly. The resulting shape of the RF coil assembly is conceived from the inventor's observation that the scan regions like the head, the shoulder and the ankle exhibit similar patterns of contours, which include a convex contour and a half tubular contour. The RF coil assembly with the concave contour portion and the half tubular contour portion is designed to conform to the scan regions with such contour patterns. Moreover, the rigid frame allows easier and faster positioning of the RF coil assembly as compared to the conventional flexible coils.

According to another embodiment of the present invention, the shape of the first rigid frame conforms to the shape of an anterior part of the top of the head and the flexible coil is bent to conform to the shape of the other portions of the anterior part of the head upon attachment of the flexible coil to the first ridge frame (3). In this way, the first rigid frame can match the anterior part of the top of the head of the subject to improve the subject's comfort and the rigidness of the frame allows easy placement of the RF coil assembly on the body regions.

According to the present invention, the flexible coil is bent in a first bending region to a substantially concave contour and in a second bending region to a substantially half tubular contour upon attaching the first end of the flexible coil to the first rigid frame. In an embodiment, the second bending region is spaced away from the first end and the first bending region extends from the first end to the second bending region.

According to another embodiment of the present invention, the first end is curved as a result of the bending along the first bending axis and the second bending axis, the second bending axis is in a proximity of the first end, the second end is curved as a result of the bending along the first bending axis, a portion of the sides of the flexible coil in the proximity of the first end is curved as a result of the bending along the second bending axis, wherein a curve bending line is formed in the flexible coil as a result of the bending along the second bending axis, the first bending region is bounded by the curved first end, the curved portion of the sides and the curve bending line, and the second bending region is bounded by the curve bending line, the straight portion of the sides and the curved second end.

According to another embodiment of the present invention, the flexible coil comprises a flexible printed circuit board, which is partially covered with foam while a first exposed portion of the flexible printed circuit board projects from the foam, said first exposed portion of the flexible printed circuit board being attached to the first rigid frame. As a result, the RF coil assembly can be assembled easily.

According to another embodiment of the present invention, the first rigid frame comprises a first part and a second part that are buckled together to retain the first exposed portion. The first part has a convex surface on a side facing the second part, a plurality of positioning studs project from the convex surface, a plurality of positioning holes are formed in the first exposed portion of the flexible printed circuit board, and the positioning studs pass through the respective positioning holes in the first exposed portion of the flexible printed circuit board, the contour of the convex surface and the distribution of the positioning studs on the convex surface and the positioning holes in the first exposed portion are configured in such a way that the flexible coil has the concave contour in the proximity of the first end and the half tubular contour in the remaining portion of the flexible coil. As a result, it is unnecessary to perform any complicated operation during assembly of the RF coil assembly.

According to another embodiment of the present invention, the RF coil assembly further comprises a second rigid frame attached to the second end of the flexible coil. The second rigid frame helps maintain the shape of the RF coil assembly and is convenient for an operator to hold the RF coil assembly.

According to another embodiment of the present invention, the RF coil assembly further comprises a control board and a pre-amplifier housed between the first part and the second part. The control board and the pre-amplifier housed within the rigid frame are not damaged easily.

According to another embodiment of the present invention, the RF coil assembly further comprises a hinge mechanism to hinge detachably the first rigid frame to a support. The hinge mechanism allows the RF coil assembly to pivot relative to the support to receive the region of the subject.

According to another embodiment of the present invention, the hinge mechanism comprises a pair of movable blocks each having a pivot shaft, a spring for acting on the movable blocks to move the movable blocks away from each other, and an operating plate for abutting against the movable blocks to move the movable blocks toward each other to retract the pivot shafts. In this way, the RF coil assembly may be detached easily from the support.

According to another embodiment of the present invention, a system is provided which comprises the RF coil assembly and a base coil assembly. The RF coil assembly is configured to be hinged detachably to the base coil assembly to form together a head coil assembly, the base coil assembly comprising a pair of support ribs for supporting the flexible coil of the RF coil assembly when the RF coil assembly is in a closed position. In this way, the RF coil assembly can be used as a top coil assembly of a head coil assembly

According to another embodiment of the present invention, the second end is flexible so that the sides of the flexible coil at the second end can be compressed toward each other to pivot the flexible coil into a space between the pair of support ribs. In this way, the flexible coil may better match a small size of the subject's head, thereby obtaining a high quality image.

According to another aspect of the present invention, it is an object to provide a MRI apparatus comprising the RF coil assembly or the system comprising the RF coil assembly and the base coil assembly.

These and other objects, features and characteristics of the present invention, as well as the methods of operation and functions of the related elements of structure and the combination of parts and economies of manufacture, will become more apparent upon consideration of the following description and the appended claims with reference to the accompanying drawings, all of which form part of this specification, wherein like reference numerals designate corresponding parts in the various figures. It is to be expressly understood, however, that the drawings are for the purpose of illustration and description only and are not intended as a definition of the limits of the invention. In any case, the scope of the invention is defined by the claims.

### BRIEF DESCRIPTION OF THE DRAWINGS

Fig. 1 is a perspective view showing a RF coil assembly according to a preferred embodiment of the present invention.
Fig. 2 is another perspective view of the RF coil assembly as shown in Fig. 1.
Fig. 3 is a perspective view showing a first part of a first rigid frame of the RF coil assembly as shown in Fig. 1.
Fig. 4 is another perspective view of the first part of the first rigid frame as shown in Fig. 3.
Fig. 5 is a perspective view showing a second part of a first rigid frame of the RF coil assembly as shown in Fig. 1.
Fig. 6 is another perspective view of the second part of the first rigid frame as shown in Fig. 5.
Fig. 7 is a perspective view showing a flexible coil of the RF coil assembly as shown in Fig. 1.
Fig. 8 is a partial perspective view showing the flexible coil attached to the first part of the first rigid frame.
Fig. 9 is a perspective view similar to FIG. 8 with a positioning pad disposed on the flexible coil.
Fig. 10 is an exploded perspective view showing a hinge mechanism of the RF coil assembly as shown in Fig. 1.
Fig. 11 is a partial cross section showing the hinge mechanism as shown in Fig. 10.
Fig. 12 is a perspective view showing the hinge mechanism as shown in Fig. 10 in a first position.
Fig. 13 is a perspective view showing the hinge mechanism as shown in Fig. 10 in a second position.
Fig. 14 is a perspective view showing the RF coil assembly as shown in Fig. 1 and a base coil assembly on which the RF coil assembly can be mounted.
Fig. 15 is a perspective view showing the RF coil assembly as shown in Fig. 1 mounted on the base coil assembly being in a closed position.
Fig. 16 is another perspective view showing the RF coil assembly as shown in Fig. 1 mounted on the base coil assembly being in a closed position.
Fig. 17 is a side view showing the RF coil assembly as shown in Fig. 1 mounted on the base coil assembly being in an opened position.
Fig. 18 is a top view showing the RF coil assembly as shown in Fig. 1, used as part of a head coil assembly.
Figs. 19 and 20 are perspective views showing the RF coil assembly well matching the head of different subjects while being used as part of a head coil assembly.
Fig. 21 is a top view showing the RF coil assembly as shown in Fig. 1, used as a shoulder coil assembly.

### DETAILED DESCRIPTION OF THE EXEMPLARY EMBODIMENTS

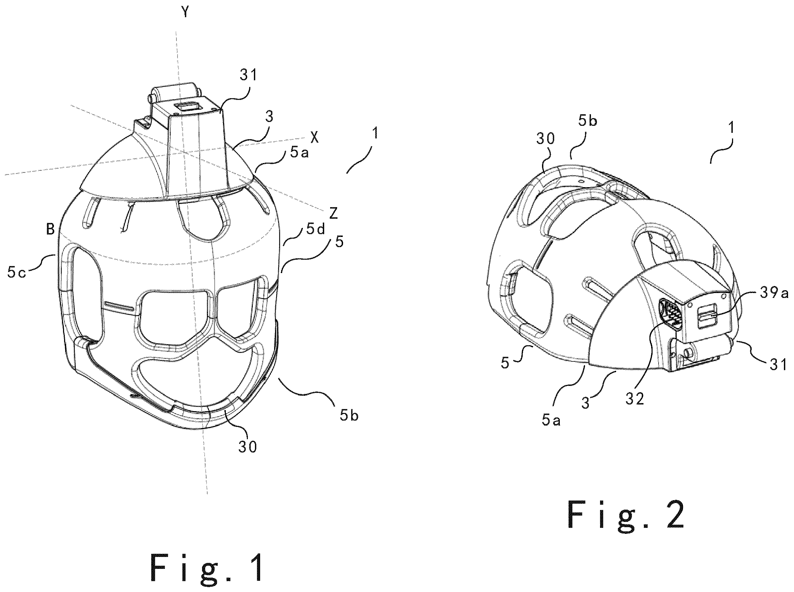

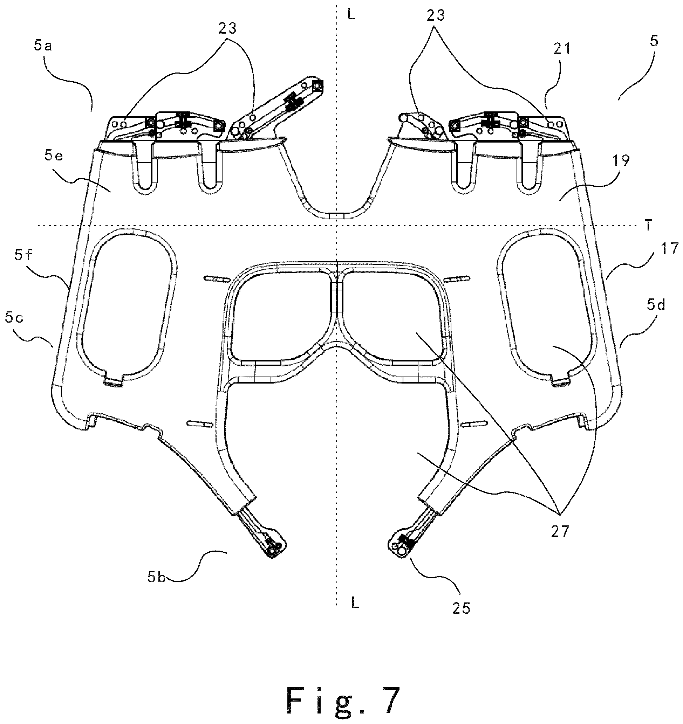

Fig. 1 is a perspective view showing a RF coil assembly according to a preferred embodiment of the present invention. Fig. 2 is another perspective view of the RF coil assembly as shown in Fig. 1. As shown in Figs 1 and 2, a RF coil assembly 1 according to the present invention comprises a first rigid frame 3 and a flexible coil 5 attached to the first rigid frame 3. Fig. 7 is a perspective view showing a flexible coil of the RF coil assembly in a naturally planar shape. The flexible coil 5 has a first end 5a, a second end 5b opposite the first end 5a, and two opposite sides 5c, 5d between the first end 5a and the second end 5b. The flexible coil 5 has a longitudinal axis L extending substantially from the first end 5a to the second end 5b and a traverse axis T perpendicular to the longitudinal axis L. The flexible coil 5 substantially extends in a plane defined by the longitudinal axis L and the traverse axis T and thus is initially in a planar shape before the flexible coil 5 is attached to the first rigid frame 3. That is, when no external force acts on the flexible coil 5, a natural shape of the flexible coil 5 is planar as shown in Fig. 7. The flexible coil 5 has an inner surface 5e configured to be placed over a region of subject and an external surface 5f opposite the inner surface 5e. The first rigid frame 3 is shaped to bend the flexible coil 5 along a first bending axis Y parallel to the longitudinal axis L and to further bend the flexible coil 5 along a second bending axis X perpendicular to the longitudinal axis L (or parallel to the traverse axis T) when the first end 5a of the flexible coil 5 is attached to the first rigid frame 3 . The bending along the first bending axis Y forms approximately a 180 degree bend, giving the initially planar flexible coil 5 a substantially half tubular shape. A bending angle produced by bending along the second bending axis X is determined to conform to any of the bending angle of the curve of the anterior part of the top of the head, for example the curve running from the top of the head to the forehead, the bending angle of the curve of the shoulder point and the bending angle of the curve of the heel. Since the bending angles of these curves have a close resemblance, the bending angle as determined above can conform to any of these curves. In addition, as the flexible coil 5 is attached to the first rigid frame 3 at the first end 5a, the second bending axis X is in a proximity of the first end 5a and only the region of the flexible coil 5 in the proximity of the first end 5a experiences the bending along the second bending axis X. As a result of the bending along the second axis X, the first end 5a is pushed inward towards the second end 5b of the flexible coil 5 to further bend a portion of the flexible coil 5 at the first end 5a so that a substantially semicircular shape is formed in the proximity of the first end 5a and a bending line B is formed in the flexible coil 5.

As such, a first bending region bounded by the curved first end 5a, the curved portion of the sides 5c, 5d of the flexible coil 5 and the bending line B is formed into a substantially concave contour as a result of the bending actions along the first and second bending axes X and Y. The concave contour conforms to the curve of the anterior part of the top of the head, the shoulder point and the heel. A second bending region bounded by the bending line B, the straight portion of the sides of the flexible coil 5 and the curved second end 5b is formed into a substantially half tubular contour as a result of the bending along the first bending axis Y. The half tubular contour conforms to the curve of the face, the upper arm and lower leg. The longitudinal length and the transverse width of the flexible coil 5 are sized such that the regions of the subject can be received in the first bending region and the second bending region. Advantageously, in accordance with the invention, the flexible coil 5 is bent into a curvature with multiple curve contours, e.g., the concave contour and the half tubular contour, to conform to the complicated curvature of the region of the subject. Moreover, the contour pattern of the concave contour and half tubular contour combination of the RF coil assembly 1 conforms to that of multiple regions of the subject, e.g., the anterior of the head, the shoulder and the ankle. Therefore, the RF coil assembly shaped into the specific curvature can be used as a multiple purpose coil.

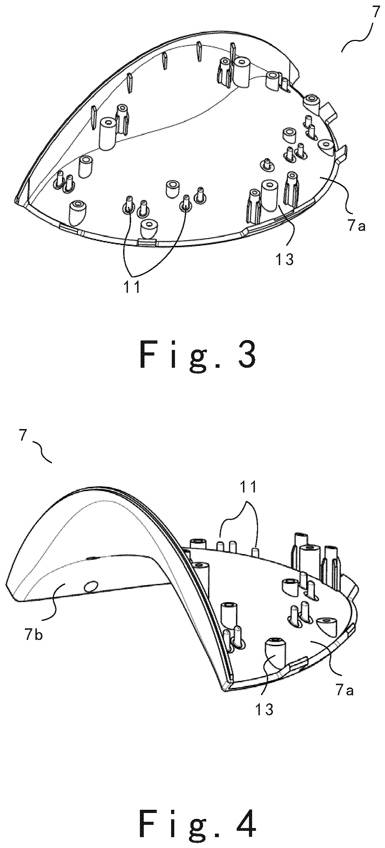

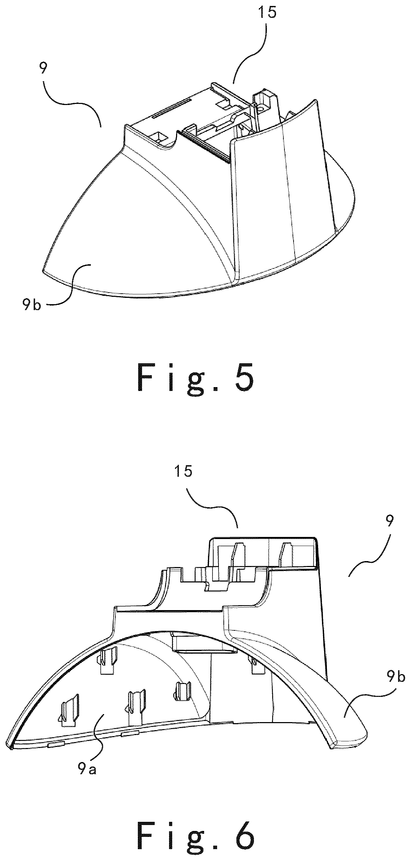

Fig. 3 is a perspective view showing a first part of a first rigid frame of the RF coil assembly as shown in Fig. 1. Fig. 4 is another perspective view of the first part of the first rigid frame as shown in Fig. 3. Fig. 5 is a perspective view showing a second part of a first rigid frame of the RF coil assembly as shown in Fig. 1. Fig. 6 is another perspective view of the second part of the first rigid frame as shown in Fig. 5. As shown in Figs. 3-6, in one embodiment, the first rigid frame 3 comprises a first part 7 and a second part 9 that are separately injection moulded. The first part 7 and the second part 9 can be buckled together to form the first rigid frame 3. The first part 7 has a concave surface 7b configured to be placed over the region of the subject and a convex surface 7a opposite the concave surface 7b. A plurality of positioning studs 11 and a plurality of threaded studs 13 project from the convex surface 7a. The second part 9 has a concave surface 9a which corresponds to the convex surface 7a of the first part 7 and a hinge mechanism seat 15 on a convex surface 9b opposite the concave surface 9a.

As the concave surface 7b is configured to be placed over the region of the subject, e.g., the anterior part of the top of the head, it can be shaped to slope downwards from the top of the head towards the forehead. As the curve of the top of the head has a close resemblance to the curve of the shoulder and posterior end of the foot, the concave surface 7b as shaped above is readily applicable to the other regions, e.g., the shoulder and the posterior end of the foot. The flexible coil 5 is attached to the corresponding convex surface 7a to form the first bending region which extends smoothly from the convex surface 7a and slopes down to the bending line B. As such, the concave surface of the first rigid frame 3 and the inner concave surface of the first bending region of the flexible coil 5, which are placed over the region of the subject, can better conform to the convex contour, e.g., the curve from the top of the head down to the forehead. The inner half tubular surface of the second bending region conforms to the half tubular contour of the face. Advantageously, the overall RF coil assembly results in the concave contour and half tubular contour which conform to the region of the subject comprised of multiple contours, e.g., the convex contour and the half tubular contour of the head, shoulder or ankle.

Alternatively, the first rigid frame 3 is shaped into half of a spherical cap to be placed over the upper top of the head. When the flexible coil 5 is attached to the convex surface 7a in the hemispherical cap shape, the flexible coil 5 is shaped into the concave bending region and the half tubular bending region. The overall RF coil assembly thereby better conforms to the region of the subject which comprises the convex contour and half tubular contour. Similarly, the concave surface 7b can also be shaped into a half bowl shape for the purpose of producing the overall RF coil assembly which better conforms to the region of the subject comprised of multiple contours . It is contemplated by those skilled in the art that the shapes described herein, e.g., the semicircular, the concave, the half tubular, the convex, the hemispherical cap, the half bowl, etc., are not precise but include the shape with a close resemblance to the described shape.

Advantageously, the overall RF coil assembly can conform to multiple regions which have similar but complex contours, e.g., the convex and the half tubular contour of the head, shoulder and ankle. Moreover, the first rigid frame 3 makes the positioning and fastening of the RF coil assembly easier and faster as compared to the conventional flexible coil. When used as the head top coil, the RF coil assembly can replace the conventional rigid head top coil to be attached directly to the conventional head base, resulting in an upgrade of the conventional rigid head coil to the head coil of the present invention with minimum cost and effort. Furthermore, the second end 5b distal to the first rigid frame 3 is still kept flexible, allowing it to be deformed to better conform to subjects of various sizes or the different regions of the subject.

Fig. 7 is a perspective view showing a flexible coil of the RF coil assembly as shown in Fig. 1. As shown in Fig 7, the flexible coil 5 is flat before being attached to the first rigid frame 3. The flexible coil 5 comprises a flexible printed circuit board 17. The flexible printed circuit board 17 is partially covered with biocompatible foam 19, while a first exposed portion 21 of the flexible printed circuit board 17 projects from the foam 19 at the first end 5a of the flexible coil 5. The foam 19 can be applied by a thermoforming method using moulds or by a layered method using foam and adhesives. There are a plurality of positioning holes 23 in the first exposed portion 21 of the flexible printed circuit board 17. In one embodiment, there is a second exposed portion 25 at a second end 5b of the flexible coil 5 opposite the first end 5a. A few openings 27 are formed in the flexible coil 5 to allow the subject's eyes, ears, nose and mouth not to be covered by the flexible coil 5, while the RF coil assembly is used as a top of the head coil assembly, thereby improving the subject's comfort.

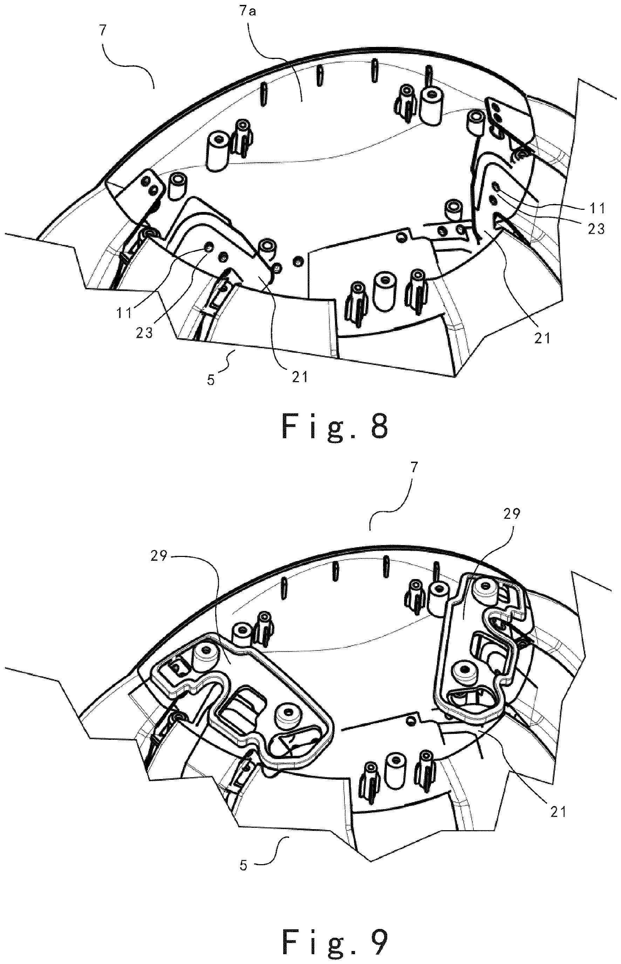

Fig. 8 is a partial perspective view showing the flexible coil attached to the first part of the first rigid frame. Fig. 9 is a perspective view similar to Fig. 8 with a positioning pad disposed on the flexible coil. When the RF coil assembly is assembled, as shown in Fig 8, the flexible coil 5 is first attached to the first part 7 of the first rigid frame 3, with the positioning studs 11 on the convex surface 7b of the first part 7 passing through the respective positioning holes 23 in the first exposed portion 21 of the flexible printed circuit board 17. The contour of the convex surface 7b and the distribution of the positioning studs 11 on the convex surface 7a of the first part 7 and the positioning holes 23 in the first exposed portion 21 are configured in such a way that the flexible coil 5 is bent into the concave contour in the proximity of the first end 5a and the half tubular contour in the remaining portion of the flexible coil after the flexible coil 5 is attached to the first part 7 of the first rigid frame 3. By means of the positioning studs 11 on the convex surface 7a of the first part 7 and the positioning holes 23 in the first exposed portion 21, it is very easy to attach the flexible coil 5 to the first part 7 and maker the flexible coil 5 have the desired contour.

A positioning pad 29 may be disposed on the first exposed portion 21 of the flexible printed circuit board 17 attached to the first part 7 to prevent the first exposed portion 21 from shifting relative to the first part 7. After other possible electronic devices, such as a control board and a pre-amplifier of the RF coil assembly, are connected with the first exposed portion 21, the second part 9 is buckled onto the first part 7. Then, the first part 7 and the second part 9 are fastened together by screws passing through the second part 9 and threaded into the threaded studs 13 on the first part 7 so that the first exposed portion 21 of the flexible printed circuit board 17 and the other possible electronic devices of the RF coil assembly are securely retained between the first part 7 and the second part 9 to form the RF coil assembly 1. The control board and the pre-amplifier are housed within the rigid frame 3 so that they are not damaged easily. Preferably, the RF coil assembly 1 may comprise a second rigid frame 30 at the second end 5b of the flexible coil 5 to securely retain the second exposed portion 25. The second rigid frame 30 may be in a handle shape so that it helps to maintain the shape of the RF coil assembly 1 and it is convenient for an operator to hold the RF coil assembly 1. Of course, it is feasible that the second rigid frame 30 is disposed at the foam-covered portion of the flexible coil 5 without the second exposed portion at the second end 5b.

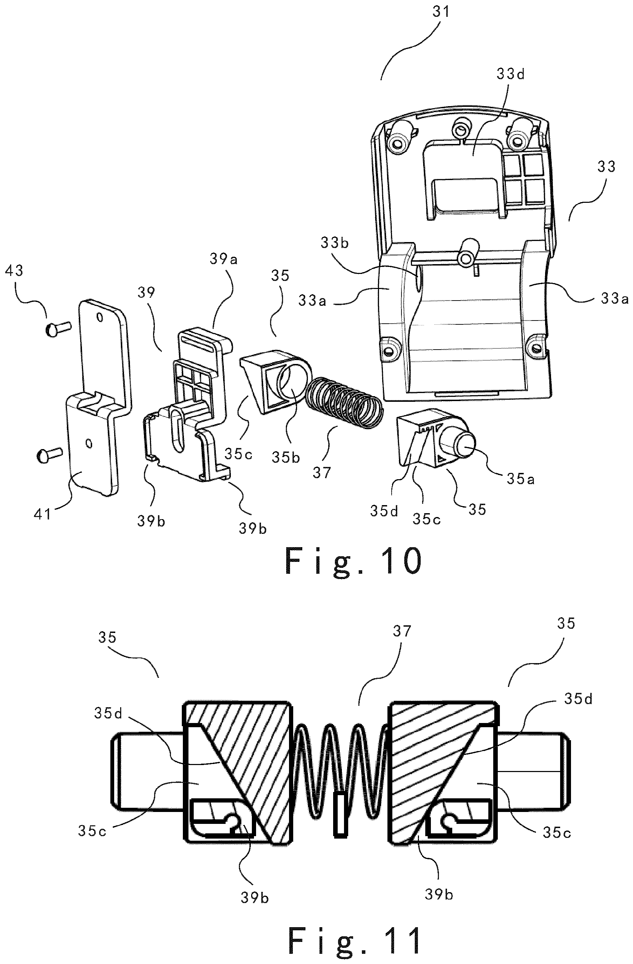

The RF coil assembly 1 according to the present invention may further comprise a retractable hinge mechanism 31 mounted onto the hinge mechanism seat 15 of the second part 9 and an interface 32 provided at the second part 9 of the first rigid frame 3 in electrical communication with the flexible coil 5. Fig. 10 is an exploded perspective view showing a hinge mechanism of the RF coil assembly as shown in Fig. 1. As shown in Fig. 10, the retractable hinge mechanism 31 comprises a bracket 33 and a pair of movable blocks 35. The bracket 33 has a pair of side plates 33a facing each other. Each of the movable blocks 35 has a pivot shaft 35a projecting from a side of the main body of the movable block and a blind hole 35b formed in the main body opposite the pivot shaft 35a. The movable blocks 35 each have a skewed slot 35c having a bevel 35d. The movable blocks 35 are disposed between the pair of side plates 33a of the bracket 33, with the pivot shaft 35a extending through a through hole 33b in the side plates 33a. Two ends of a spring 37 are received within the blind hole 35b of the movable blocks 35 so that the movable blocks 35 always move away from each other. The hinge mechanism 31 further comprises an operating plate 39 having an operating button 39a formed at one end and two projections 39b at the other end. FIG. 11 is a partial cross section showing the hinge mechanism as shown in FIG. 10. After the operating plate 39 is mounted in place by a fixing plate 41 which is fastened onto the bracket 33 by screws 43, the operating button 39a of the operating plate 39 extends through an opening 33d in the bracket 33 to be accessible from outside and two projections 39b extend into the skewed slot 35c of the respective movable blocks 35 to abut against the bevel 35d, as shown in Fig. 11.

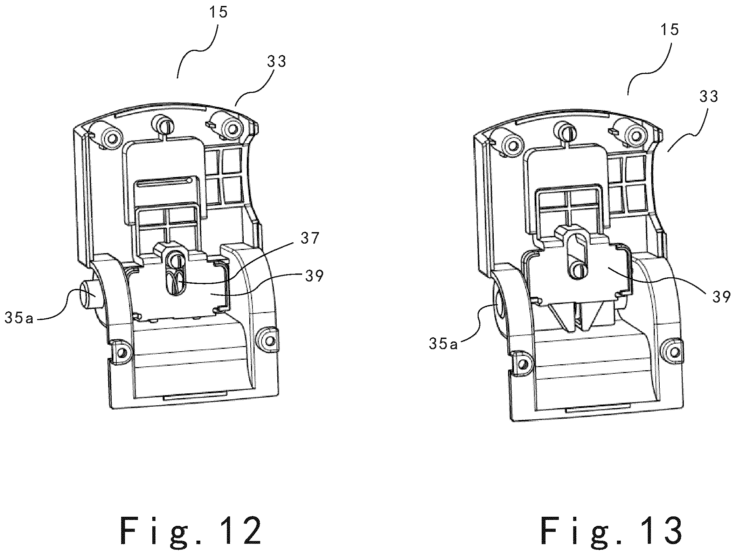

Fig. 12 is a perspective view showing the hinge mechanism as shown in Fig. 10 in a first position. Fig. 13 is a perspective view showing the hinge mechanism as shown in Fig. 10 in a second position. In a usual state, the pivot shaft 35a of each movable block 35 projects from the through hole 33b in the side plates 33a under an action of the spring 37, as shown in Fig. 12. When the operating button 39a of the operating plate 39 is pulled upward relative to the bracket 33, the two projections 39b abut against the bevel 35d of the respective movable blocks 35 and move the movable blocks 35 toward each other so that the pivot shaft 35a of each movable block 35 retracts relative to the bracket 33, as shown in Fig. 13. When the operating button 39a of the operating plate 39 is released, the spring 37 pushes the pivot shafts 35a of the movable blocks 35 to project from the through hole 33b in the side plates 33a again, as shown in Fig. 12. It should be understood that the hinge mechanism 31 can be implemented in other ways. For example, the movable blocks 35 and the operating plate 39 may be configured in such a way that the pivot shaft 35a of each movable block 35 retracts relative to the bracket 33 when the operating plate 39 moves downward relative to the bracket 33. Further, the bracket 33 may be omitted and a portion of the first rigid frame 3 may function as the bracket 33.

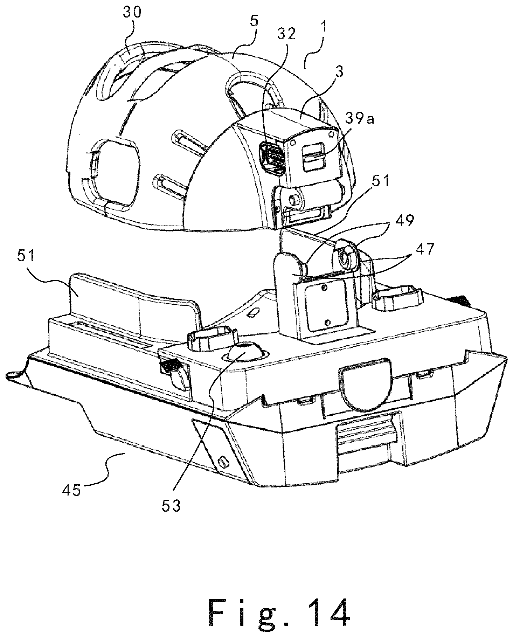

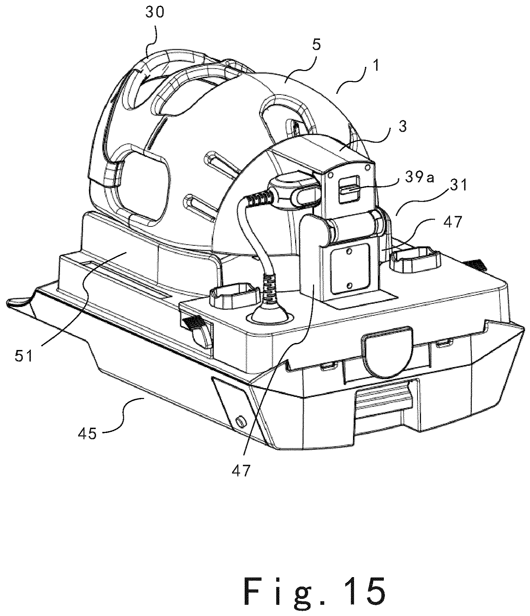

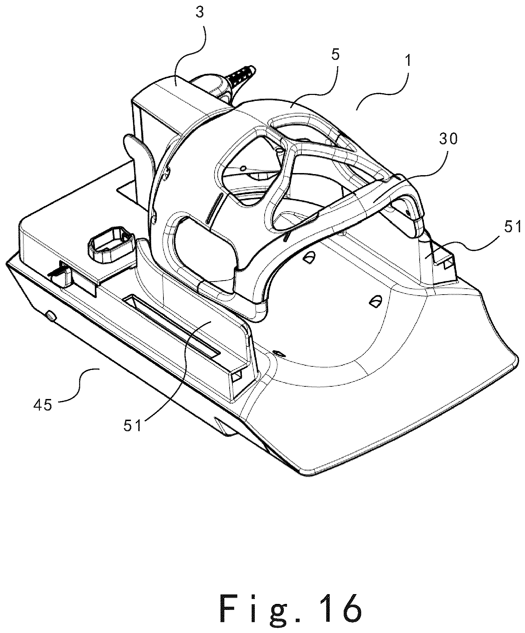

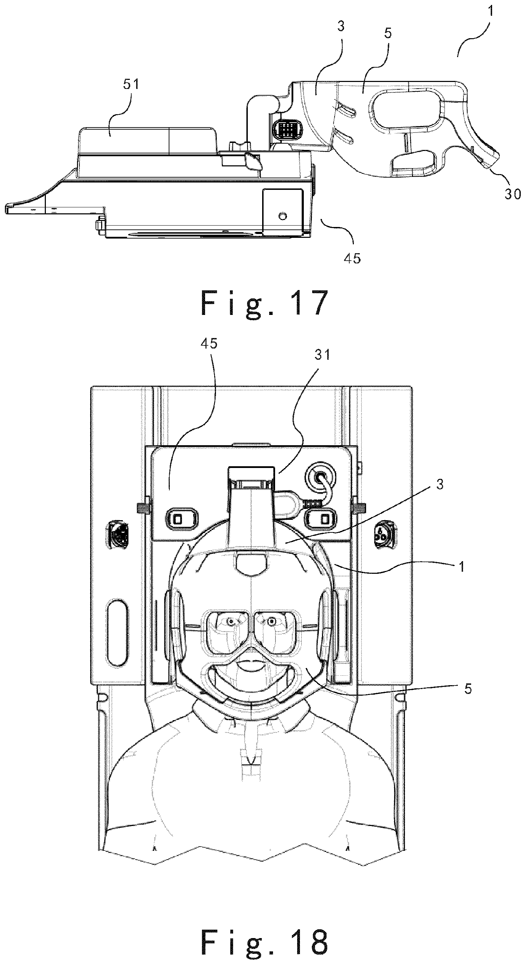

Fig. 14 is a perspective view showing the RF coil assembly as shown in Fig. 1 and a base coil assembly on which the RF coil assembly can be mounted. The base coil assembly 45 is configured to support the subject's head when the subject's head is imaged and comprises a coil disposed within a rigid housing. There are a pair of support arms 47 on the base coil assembly 45. A hole 49 for receiving the pivot shaft 35a of the movable block 35 is formed in each of the support arms 47 so that the RF coil assembly 1 may be mounted detachably onto the base coil assembly 45. Specifically, when it is necessary to mount the RF coil assembly 1 onto the base coil assembly 45, the operating button 39a of the operating plate 39 is pulled upward so that the pivot shaft 35a of each movable block 35 retracts. While the pivot shaft 35a of each movable block 35 is in alignment with the hole 49 in the support arms 47, the operating button 39a of the operating plate 39 is released and the pivot shaft 35a extends into the respective hole 49. As a result, the RF coil assembly 1 is mounted pivotally onto the base coil assembly 45 to form together a head coil assembly. When the operating button 39a of the operating plate 39 is pulled upward again, the RF coil assembly 1 is detachable from the base coil assembly 45. There are a pair of support ribs 51 on the base coil assembly 45. The support ribs 51 support the flexible coil 5 of the RF coil assembly 1 when the RF coil assembly 1 is in a closed position, as shown in Figs. 15 and 16. There is an interface 53 in electrical communication with an Analog to Digital Converter of a MRI apparatus. Fig. 15 is a perspective view showing the RF coil assembly as shown in Fig. 1 mounted on the base coil assembly 45 being in a closed position. Fig. 16 is another perspective view showing the RF coil assembly as shown in Fig. 1 mounted on the base coil assembly 45 being in a closed position. Fig. 17 is a side view showing the RF coil assembly as shown in Fig. 1 mounted on the base coil assembly 45 being in an opened position. By grasping the second rigid frame 30, it is very easy for the operator to pivot the RF coil assembly from the closed position to the opened position or from the opened position to the closed position. Fig. 18 is a top view showing the RF coil assembly 1 used as a part of a head top coil assembly to scan the subject's head. It should be understood that the RF coil assembly 1 can be detachably attached to another support such as a subject support couch.

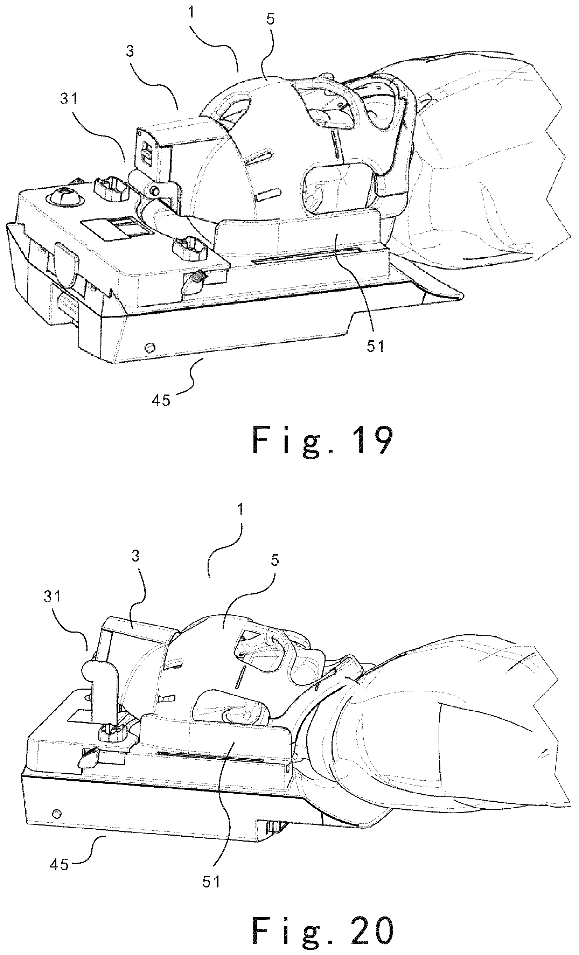

Figs. 19 and 20 are perspective views showing the RF coil assembly matching the head of different subjects while being used as a part of a head top coil assembly. Generally, in the closed position, the flexible coil 5 of the RF coil assembly 1 is supported on the support ribs 51 on the base coil assembly 45 to match the shape and size of a majority of subjects. However, as shown in Fig. 19, since the second end 5b always has flexibility, by compressing the sides 5c, 5d of the flexible coil 5 at the second end 5b toward each other and pivoting the flexible coil 5 down into a space between the support ribs 51, the flexible coil 5 may better match a subject's head of a small size, thereby obtaining a high quality image. Compared with Fig. 19, the flexible coil 5 as shown in Fig. 20 is pivoted down much more to match a child's head.

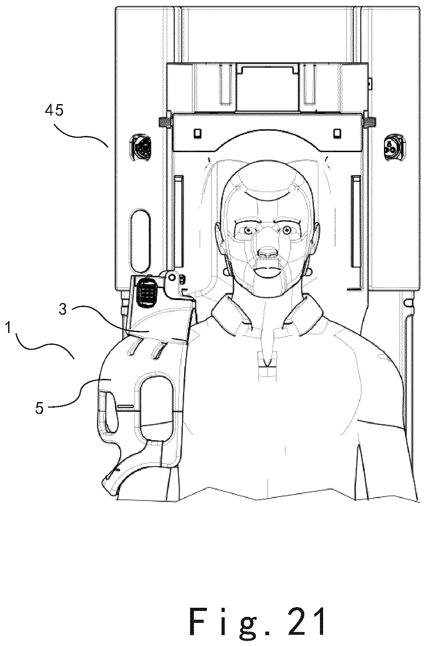

Fig. 21 is a top view showing the RF coil assembly as shown in FIG. 1 used as a shoulder coil assembly. As shown in Fig. 21, the RF coil assembly 1 is detached from the base coil assembly 45 and used as a shoulder coil assembly. The RF coil assembly 1 according to the present invention also matches well a subject's shoulder. Of course, the RF coil assembly 1 also matches well a subject's ankle and thus may be used as an ankle coil assembly.

Although the invention has been described in detail for the purpose of illustration, it is to be understood that such detail is solely for that purpose and that the invention is not limited to the disclosed embodiments, but, on the contrary, is intended to cover modifications that are within the scope of the appended claims.

## Claims

1. A RF coil assembly (1) for MRI comprising:
- a first rigid frame (3); and
- a flexible coil (5) having a first end (5a), a second end (5b) opposite the first end (5a) and a longitudinal axis (L) extending from the first end (5a) to the second end (5b), the flexible coil (5) being in a naturally planar shape before the flexible coil (5) is attached to the first rigid frame (3), wherein the first end (5a) is attached to the first rigid frame (3) and the second end (5b) is away from the first rigid frame (3) after assembly of the RF coil assembly is finished, wherein the first rigid frame (3) is shaped to bend the flexible coil (5) upon attaching the first end (5a) of the flexible coil (5) to the first rigid frame (3) to form a shape of the RF coil assembly (1) which conforms to a shape of a region of a subject with multiple contours, wherein the flexible coil is bent into a curvature with multiple curve contours, wherein the first rigid frame is shaped to bend the flexible coil along a first bending axis parallel to the longitudinal axis and to further bend the flexible coil along a second bending axis perpendicular to the longitudinal axis (L) when the first end of the flexible coil is attached to the first rigid frame and the bending along the first bending axis forms approximately a 180 degree bend, giving the initially planar flexible coil a substantially half tubular shape, and the bending along the second bending axis extends over a bending angle of the subject's outer surface's curvature determined in conformity to the shape of the region of the subject, wherein only a region of the flexible coil in proximity of the first end experiences the bending along the second bending axis, thereby forming a first bending region having a concave contour.

2. The RF coil assembly (1) according to claim 1, wherein the shape of the RF coil assembly (1) comprises the concave contour that conforms to a convex contour of the region of the subject and a half tubular contour that conforms to a half tubular contour of the region of the subject.

3. The RF coil assembly (1) according to claim 1, wherein the region of the subject comprises any one of the anterior part of the head, shoulder and ankle.

4. The RF coil assembly (1) according to claim 1, wherein the first rigid frame (3) is in a shape conforming to a shape of an anterior part of the top of the head and the flexible coil (5) is bent to form a shape conforming to a shape of the other portions of the anterior part of the head upon attachment to the first rigid frame (3)..

5. The RF coil assembly (1) according to claim 1, wherein the second bending region is spaced away from the first end (5a) and the first bending region extends from the first end (5a) to the second bending region.

6. The RF coil assembly (1) according to claim 5, wherein the first end (5a) is curved as a result of the bending along the first bending axis (Y) and the second bending axis (X), wherein the second bending axis (X) is in a proximity of the first end (5a), the second end (5b) is curved as a result of the bending along the first bending axis (Y), a portion of the sides (5c, 5d) of the flexible coil (5) in the proximity of the first end (5a) is curved as a result of the bending along the second bending axis (X), wherein a curve bending line (B) is formed in the flexible coil (5) as a result of the bending along the second bending axis (X), the first bending region is bounded by the curved first end (5a), the curved portion of the sides (5c, 5d) and the curve bending line (B), and the second bending region is bounded by the curve bending line (B), a straight portion of the sides (5c, 5d) and the curved second end (5b).

7. The RF coil assembly (1) according to claim 1, wherein, the flexible coil (5) comprises a flexible printed circuit board (17), the flexible printed circuit board (17) is partially covered with foam (19) while a first exposed portion (21) of the flexible printed circuit board (17) projects from the foam (19), the first exposed portion (21) of the flexible printed circuit board (17) is attached to the first rigid frame (3).

8. The RF coil assembly (1) according to claim 7, wherein the first rigid frame (3) comprises a first part (7) and a second part (9) that are buckled together to retain the first exposed portion (21), the first part (7) has a convex surface (7b) on a side facing the second part (9), a plurality of positioning studs (11) project from the convex surface (7b), a plurality of positioning holes (23) are formed in the first exposed portion (21) of the flexible printed circuit board (17), the positioning studs (11) pass through the respective positioning holes (23) in the first exposed portion (21) of the flexible printed circuit board (17), the contour of the convex surface (7b) and the distribution of the positioning studs (11) on the convex surface (7b) and the positioning holes (23) in the first exposed portion (21) are configured in such a way that the flexible coil (5) has the concave contour in the proximity of the first end (5a) and a half tubular contour in the remaining portion of the flexible coil.

9. The RF coil assembly (1) according to claim 1, further comprising a second rigid frame (30) attached to the second end (5b) of the flexible coil (5).

10. The RF coil assembly (1) according to claim 7, further comprising a control board and a pre-amplifier housed between the first part (7) and the second part (9).

11. The RF coil assembly (1) according to claim 1, further comprising a hinge mechanism (31) to hinge detachably the first rigid frame (3) to a support.

12. The RF coil assembly (1) according to claim 11, wherein the hinge mechanism (31) comprises a pair of movable blocks (35) each having a pivot shaft (35a), a spring (37) for acting on the movable blocks (35) to move the movable blocks (35) away from each other, and an operating plate (39) for abutting against the movable blocks (35) to move the movable blocks (35) toward each other to retract the pivot shafts (35a).

13. A system comprising the RF coil assembly (1) according to claim 11 and a base coil assembly (45), wherein the RF coil assembly (1) is configured to be hinged detachably to the base coil assembly (45) to form together a head coil assembly, the base coil assembly (45) comprises a pair of support ribs (51) for supporting the flexible coil (5) of the RF coil assembly (1) when the RF coil assembly (1) is in a closed position.

14. The system according to claim 13, wherein the second end (5b) of the RF coil assembly (1) is configured to have flexibility so that the sides (5c, 5d) of the flexible coil (5) at the second end (5b) can be compressed toward each other to pivot the flexible coil (5) into a space between the pair of support ribs (51).

15. A MRI apparatus comprising a RF coil assembly according to any one of claims 1-12 or a system according to any one of claims 13-14.

## Patentansprüche

1. HF-Spulenanordnung (1) für MRT, umfassend:
- einen ersten starren Rahmen (3); und
- eine flexible Spule (5) mit einem ersten Ende (5a), einem dem ersten Ende (5a) gegenüberliegenden zweiten Ende (5b) und einer Längsachse (L), welche sich von dem ersten Ende (5a) zu dem zweiten Ende (5b) erstreckt, wobei die flexible Spule (5) eine natürlich ebene Form aufweist, bevor die flexible Spule (5) an dem ersten starren Rahmen (3) befestigt wird, wobei das erste Ende (5a) an dem ersten starren Rahmen (3) befestigt ist und das zweite Ende (5b) von dem ersten starren Rahmen (3) entfernt ist, nachdem die Montage der HF-Spulenanordnung abgeschlossen ist,
wobei der erste starre Rahmen (3) so geformt ist, dass er die flexible Spule (5) beim Befestigen des ersten Endes (5a) der flexiblen Spule (5) an dem ersten starren Rahmen (3) biegt, um eine Form der HF-Spulenanordnung (1) zu bilden, welche einer Form eines Bereichs eines Subjekts mit mehreren Konturen entspricht, wobei die flexible Spule in eine Krümmung mit mehreren Kurvenkonturen gebogen wird, wobei der erste starre Rahmen so geformt ist, dass er die flexible Spule entlang einer ersten Biegeachse parallel zu der Längsachse biegt und die flexible Spule weiter entlang einer zweiten Biegeachse senkrecht zu der Längsachse (L) biegt, wenn das erste Ende der flexiblen Spule an dem ersten starren Rahmen befestigt ist, und die Biegung entlang der ersten Biegeachse ungefähr eine 180-Grad-Biegung bildet, wodurch die anfänglich ebene flexible Spule eine im Wesentlichen halbröhrenförmige Form erhält, und die Biegung entlang der zweiten Biegeachse sich über einen Biegewinkel der Krümmung der Außenfläche des Subjekts erstreckt, welcher in Übereinstimmung mit der Form des Bereichs des Subjekts bestimmt wird, wobei nur ein Bereich der flexiblen Spule in der Nähe des ersten Endes die Biegung entlang der zweiten Biegeachse erfährt, wodurch ein erster Biegebereich mit einer konkaven Kontur gebildet wird.

2. HF-Spulenanordnung (1) nach Anspruch 1, wobei die Form der HF-Spulenanordnung (1) die konkave Kontur, die einer konvexen Kontur des Bereichs des Subjekts entspricht, und eine halbröhrenförmige Kontur umfasst, die einer halbröhrenförmigen Kontur des Bereichs des Subjekts entspricht.

3. HF-Spulenanordnung (1) nach Anspruch 1, wobei der Bereich des Subjekts den vorderen Teil des Kopfes, die Schulter oder den Knöchel umfasst.

4. HF-Spulenanordnung (1) nach Anspruch 1, wobei der erste starre Rahmen (3) eine Form aufweist, welche der Form eines vorderen Teils der Oberseite des Kopfes entspricht, und die flexible Spule (5) gebogen ist, um nach der Befestigung an dem ersten starren Rahmen (3) eine Form anzunehmen, die der Form der anderen Abschnitte des vorderen Teils des Kopfes entspricht.

5. HF-Spulenanordnung (1) nach Anspruch 1, wobei der zweite Biegebereich von dem ersten Ende (5a) beabstandet ist und der erste Biegebereich sich von dem ersten Ende (5a) zu dem zweiten Biegebereich erstreckt.

6. HF-Spulenanordnung (1) nach Anspruch 5, wobei das erste Ende (5a) infolge der Biegung entlang der ersten Biegeachse (Y) und der zweiten Biegeachse (X) gekrümmt ist,
wobei die zweite Biegeachse (X) in einer Nähe des ersten Endes (5a) liegt, das zweite Ende (5b) infolge der Biegung entlang der ersten Biegeachse (Y) gekrümmt ist, ein Abschnitt der Seiten (5c, 5d) der flexiblen Spule (5) in der Nähe des ersten Endes (5a) infolge der Biegung entlang der zweiten Biegeachse (X) gekrümmt ist, wobei in der flexiblen Spule (5) infolge der Biegung entlang der zweiten Biegeachse (X) eine Kurvenbiegelinie (B) gebildet wird, der erste Biegebereich durch das gekrümmte erste Ende (5a), den gekrümmten Abschnitt der Seiten (5c, 5d) und die Kurvenbiegelinie (B) begrenzt wird, und der zweite Biegebereich durch die Kurvenbiegelinie (B), einen geraden Abschnitt der Seiten (5c, 5d) und das gekrümmte zweite Ende (5b) begrenzt wird.

7. HF-Spulenanordnung (1) nach Anspruch 1, wobei die flexible Spule (5) eine flexible Leiterplatte (17) umfasst, die flexible Leiterplatte (17) teilweise mit Schaumstoff (19) bedeckt ist, während ein erster freiliegender Abschnitt (21) der flexiblen Leiterplatte (17) aus dem Schaumstoff (19) vorsteht, der erste freiliegende Abschnitt (21) der flexiblen Leiterplatte (17) an dem ersten starren Rahmen (3) befestigt ist.

8. HF-Spulenanordnung (1) nach Anspruch 7, wobei der erste starre Rahmen (3) einen ersten Teil (7) und einen zweiten Teil (9) umfasst, welche zusammengeschnallt sind, um den ersten freiliegenden Abschnitt (21) festzuhalten, der erste Teil (7) eine konvexe Oberfläche (7b) auf einer dem zweiten Teil (9) zugewandten Seite aufweist, eine Vielzahl von Positionierungsbolzen (11) von der konvexen Oberfläche (7b) vorstehen, eine Vielzahl von Positionierungslöchern (23) in dem ersten freiliegenden Abschnitt (21) der flexiblen Leiterplatte (17) gebildet sind, die Positionierungsbolzen (11) durch die jeweiligen Positionierungslöcher (23) in dem ersten freiliegenden Abschnitt (21) der flexiblen Leiterplatte (17) verlaufen, die Kontur der konvexen Oberfläche (7b) und die Verteilung der Positionierungsbolzen (11) auf der konvexen Oberfläche (7b) und die Positionierungslöcher (23) in dem ersten freiliegenden Abschnitt (21) so konfiguriert sind, dass die flexible Spule (5) die konkave Kontur in der Nähe des ersten Endes (5a) und eine halbröhrenförmige Kontur in dem verbleibende Teil der flexiblen Spule aufweist.

9. HF-Spulenanordnung (1) nach Anspruch 1, welche weiter einen zweiten starren Rahmen (30) umfasst, welcher an dem zweiten Ende (5b) der flexiblen Spule (5) befestigt ist.

10. HF-Spulenanordnung (1) nach Anspruch 7, welche weiter eine Steuerplatine und einen Vorverstärker umfasst, welche zwischen dem ersten Teil (7) und dem zweiten Teil (9) untergebracht sind.

11. HF-Spulenanordnung (1) nach Anspruch 1, welche weiter einen Scharniermechanismus (31) umfasst, um den ersten starren Rahmen (3) lösbar an einer Stütze einzuhängen.

12. HF-Spulenanordnung (1) nach Anspruch 11, wobei der Scharniermechanismus (31) ein Paar beweglicher Blöcke (35) mit jeweils einer Schwenkwelle (35a), einer Feder (37) zum Einwirken auf die beweglichen Blöcke (35), um die beweglichen Blöcke (35) voneinander weg zu bewegen, und einer Betätigungsplatte (39) zum Anstoßen an die beweglichen Blöcke (35), um die beweglichen Blöcke (35) aufeinander zu zu bewegen und so die Schwenkwellen (35a) zurückzuziehen, umfasst.

13. System, welches die HF-Spulenanordnung (1) nach Anspruch 11 und eine Basisspulenanordnung (45) umfasst, wobei die HF-Spulenanordnung (1) so konfiguriert ist, dass sie lösbar an der Basisspulenanordnung (45) angebracht werden kann, um zusammen eine Kopfspulenanordnung zu bilden, die Basisspulenanordnung (45) ein Paar Stützrippen (51) zum Stützen der flexiblen Spule (5) der HF-Spulenanordnung (1) umfasst, wenn sich die HF-Spulenanordnung (1) in einer geschlossenen Position befindet.

14. System nach Anspruch 13, wobei das zweite Ende (5b) der HF-Spulenanordnung (1) so konfiguriert ist, dass es Flexibilität aufweist, sodass die Seiten (5c, 5d) der flexiblen Spule (5) an dem zweiten Ende (5b) aufeinander zu gedrückt werden können, um die flexible Spule (5) in einen Raum zwischen dem Paar Stützrippen (51) zu schwenken.

15. MRT-Einrichtung, welche eine HF-Spulenanordnung nach einem der Ansprüche 1-12 oder ein System nach einem der Ansprüche 13-14 umfasst.

## Revendications

1. Ensemble bobine RF (1) pour une IRM comprenant :
- un premier cadre rigide (3) ; et
- une bobine flexible (5) présentant une première extrémité (5a), une seconde extrémité (5b) opposée à la première extrémité (5a) et un axe longitudinal (L) s'étendant depuis la première extrémité (5a) jusqu'à la seconde extrémité(5b), la bobine flexible (5) présentant une forme naturellement plane avant que la bobine flexible (5) ne soit fixée au premier cadre rigide (3), dans lequel la première extrémité (5a) est fixée au premier cadre rigide (3) et la seconde extrémité (5b) est éloignée du premier cadre rigide (3) une fois l'assemblage de l'ensemble bobine RF terminé,
dans lequel le premier cadre rigide (3) est façonné pour plier la bobine flexible (5) lors de la fixation de la première extrémité (5a) de la bobine flexible (5) au premier cadre rigide (3) pour former une forme de l'ensemble bobine RF (1) qui épouse une forme d'une région d'un sujet avec de multiples contours, dans lequel la bobine flexible est pliée selon une courbure avec de multiples contours de courbe, dans lequel le premier cadre rigide est façonné pour plier la bobine flexible le long d'un premier axe de courbure parallèle à l'axe longitudinal et pour plier davantage la bobine flexible le long d'un second axe de courbure perpendiculaire à l'axe longitudinal (L) lorsque la première extrémité de la bobine flexible est fixée au premier cadre rigide et la courbure le long du premier axe de courbure forme approximativement une courbure de 180 degrés, donnant à la bobine flexible initialement plane une forme sensiblement semi-tubulaire, et la courbure le long du second axe de courbure s'étend sur un angle de courbure de la courbure de la surface extérieure du sujet déterminé conformément à la forme de la région du sujet, dans lequel seulement une région de la bobine flexible à proximité de la première extrémité subit la courbure le long du second axe de courbure, ce qui permet de former une première région de courbure présentant un contour concave.

2. Ensemble bobine RF (1) selon la revendication 1, dans lequel la forme de l'ensemble bobine RF (1) comprend le contour concave qui épouse un contour convexe de la région du sujet et un contour semi-tubulaire qui épouse un contour semi-tubulaire de la région du sujet.

3. Ensemble bobine RF (1) selon la revendication 1, dans lequel la région du sujet comprend l'une quelconque de la partie antérieure de la tête, de l'épaule et de la cheville

4. Ensemble bobine RF (1) selon la revendication 1, dans lequel le premier cadre rigide (3) présente une forme épousant une forme d'une partie antérieure du sommet de la tête et la bobine flexible (5) est pliée pour former un forme épousant une forme des autres parties de la partie antérieure de la tête lors de la fixation au premier cadre rigide (3).

5. Ensemble bobine RF (1) selon la revendication 1, dans lequel la seconde région de courbure est espacée de la première extrémité (5a) et la première région de courbure s'étend depuis la première extrémité (5a) jusqu'à la seconde région de courbure.

6. Ensemble bobine RF (1) selon la revendication 5, dans lequel la première extrémité (5a) est cintrée à la suite de la courbure le long du premier axe de courbure (Y) et du second axe de courbure (X),
dans lequel le second axe de courbure (X) se trouve à proximité de la première extrémité (5a), la seconde extrémité (5b) est cintrée à la suite de la courbure le long du premier axe de courbure (Y), une partie des côtés (5c , 5d) de la bobine flexible (5) à proximité de la première extrémité (5a) est cintrée à la suite de la courbure le long du second axe de courbure (X), dans lequel une ligne de courbure de courbe (B) est formée dans la bobine flexible (5) à la suite de la courbure le long du second axe de courbure (X), la première région de courbure est délimitée par la première extrémité cintrée (5a), la partie cintrée des côtés (5c, 5d) et la ligne de courbure de courbe (B), et la seconde région de courbure est délimitée par la ligne de courbure de courbe (B), une partie droite des côtés (5c, 5d) et la seconde extrémité cintrée (5b).

7. Ensemble bobine RF (1) selon la revendication 1, dans lequel la bobine flexible (5) comprend une carte de circuit imprimé flexible (17), la carte de circuit imprimé flexible (17) est partiellement recouverte de mousse (19) tandis qu'une première partie exposée (21) de la carte de circuit imprimé flexible (17) fait saillie à partir de la mousse (19), la première partie exposée (21) de la carte de circuit imprimé flexible (17) est fixée au premier cadre rigide (3).

8. Ensemble bobine RF (1) selon la revendication 7, dans lequel le premier cadre rigide (3) comprend une première partie (7) et une seconde partie (9) qui sont nouées ensemble pour retenir la première partie exposée (21), la première partie (7) présente une surface convexe (7b) sur un côté faisant face à la seconde partie (9), une pluralité de plots de positionnement (11) font saillie depuis la surface convexe (7b), une pluralité de trous de positionnement (23) sont formés dans la première partie exposée (21) de la carte de circuit imprimé flexible (17), les plots de positionnement (11) passent à travers les trous de positionnement respectifs (23) dans la première partie exposée (21) de la carte de circuit imprimé flexible (17), le contour de la surface convexe (7b) et la répartition des plots de positionnement (11) sur la surface convexe (7b) et les trous de positionnement (23) dans la première partie exposée (21) sont configurés de telle manière que la bobine flexible (5) présente le contour concave à proximité de la première extrémité (5a) et un contour semi-tubulaire dans la partie restante de la bobine flexible.

9. Ensemble bobine RF(1) selon la revendication 1, comprenant en outre un second cadre rigide (30) fixé à la seconde extrémité (5b) de la bobine flexible (5).

10. Ensemble bobine RF (1) selon la revendication 7, comprenant en outre une carte de commande et un préamplificateur logé entre la première partie (7) et la seconde partie (9).

11. Ensemble bobine RF (1) selon la revendication 1, comprenant en outre un mécanisme d'articulation (31) pour articuler de manière amovible le premier cadre rigide (3) à un support.

12. Ensemble bobine RF (1) selon la revendication 11, dans lequel le mécanisme d'articulation (31) comprend une paire de blocs mobiles (35) présentant chacun un arbre de pivotement (35a), un ressort (37) pour agir sur les blocs mobiles (35) pour éloigner les blocs mobiles (35) les uns des autres, et une plaque de fonctionnement (39) destinée à venir en butée contre les blocs mobiles (35) pour déplacer les blocs mobiles (35) les uns vers les autres pour rétracter les arbres de pivotement (35a).

13. Système comprenant l'ensemble bobine RF (1) selon la revendication 11 et un ensemble bobine de base (45), dans lequel l'ensemble bobine RF (1) est configuré pour être articulé de manière amovible à l'ensemble bobine de base (45) pour former ensemble un ensemble bobine de tête, l'ensemble bobine de base (45) comprend une paire de nervures de support (51) pour supporter la bobine flexible (5) de l'ensemble bobine RF (1) lorsque l'ensemble bobine RF (1) est dans une position fermée.

14. Système selon la revendication 13, dans lequel la seconde extrémité (5b) de l'ensemble bobine RF (1) est configurée pour présenter une flexibilité de telle sorte que les côtés (5c, 5d) de la bobine flexible (5) au niveau de la seconde extrémité (5b) puissent être compressés l'un vers l'autre pour faire pivoter la bobine flexible (5) dans un espace entre la paire de nervures de support (51).

15. Appareil IRM comprenant un ensemble bobine RF selon l'une quelconque des revendications 1-12 ou un système selon l'une quelconque des revendications 13-14.
